Europäisches Patentamt

European Patent Office

Office européen des brevets

(11) Publication number: **0 224 543**
**B1**

## (12) EUROPEAN PATENT SPECIFICATION

(45) Date of publication of patent specification: 16.08.90

(21) Application number: **86903490.0**

(22) Date of filing: **30.05.86**

(86) International application number:
**PCT/GB86/00302**

(87) International publication number:
**WO 86/06949 04.12.86 Gazette 86/26**

(51) Int. Cl.⁵: **A 61 B 5/11, A 61 B 8/00**

## (54) SWAY MONITOR.

(30) Priority: **01.06.85 GB 8513867**
**06.09.85 GB 8522170**

(43) Date of publication of application:
**10.06.87 Bulletin 87/24**

(45) Publication of the grant of the patent:
**16.08.90 Bulletin 90/33**

(84) Designated Contracting States:
**BE CH DE FR GB IT LI NL**

(56) References cited:
**EP-A-0 064 788**
**WO-A-86/03392**
**FR-A-2 198 641**
**FR-A-2 571 955**
**US-A-3 924 450**

(73) Proprietor: **BEECHAM GROUP PLC**
**Beecham House Great West Road**
**Brentford Middlesex TW8 9BD (GB)**

(72) Inventor: **FRANCIS, Graham, Russell**
**19 Penshurst Park Hill, Old Harlow**
**Essex CM17 OBP (GB)**
Inventor: **PETTITT, Keith, Edward, Ernest**
**22 Markwells Elsenham, Bishop's Stortford**
**Hertfordshire CM22 6LT (GB)**
Inventor: **McCLELLAND, Graham, Ralph**
**25 The Barons Bishop's Stortford**
**Hertfordshire CM23 4HR (GB)**
Inventor: **BATES, Colin, David**
**Newhaven Cottage Star Green, Whiteshill**
**Stroud Gloucestershire GL6 6AD (GB)**

(74) Representative: **Lockwood, Barbara Ann et al**
**Beecham Pharmaceuticals Biosciences Research**
**Centre Great Burgh Yew Tree Bottom Road**
**Epsom Surrey KT18 5XQ (GB)**

Courier Press, Leamington Spa, England.

## Description

This invention relates to a sway detecting and monitoring apparatus, particularly to such an apparatus for use in assessing drug effects on human volunteers or patients.

The upright stance in the human is an unstable position and therefore always accompanied by involuntary motion. This postural sway is a reflection of the noise and regulatory activity of the several control loops involved in the maintenance of balance. The principal sensory systems involved in this are proprioception, the vestibular system and vision, and the afferent neuronal pathways within the central nervous system. The afferent and efferent neuronal pathways involve spinal cord, brainstem, cerebellum, midbrain and sensorimotor cortex. Interference in any of these systems can therefore affect balance, and body sway is often used an an index of psychotropic drug activity in normal subjects. It has also been proposed as an objective index of the extent of progression of, and drug effect on, diffuse brain disease such as dementia [Visser, H. (1983) Age and Ageing, *12,* 296—301].

Numerous methods have been used to obtain a measure of body sway, and have included: the attachment of a thread with weight and pointer to the subjects clothing, the use of pulleys, the employment of balance platforms that resolve sway components from vertically applied forces, the use of cine photography or more recently video recordings, doppler radar ranging, and collimated light sources with linear photodiode arrays. Most of these methods are expensive, cumbersome and require complex preparatory procedures before useful results can be obtained.

Ultrasound has been used previously to measure axial eye movement [Haines, J. (1977) Ultrasound in Med. Biol., *3,* 39—45], and facial movement [Haines, J. (1983) Med. Biol. Eng. Comput., *21,* 105—106].

The present invention provides an ultrasound technique for measuring and monitoring body sway.

According to the present invention there is provided a sway detecting and monitoring device comprising

a) two or more ultrasound transmitters arranged to direct a respective ultrasound pulse across a subject area amd generate a first timing signal when the pulse is transmitted

b) an ultrasound receiver associated with each transmitter to detect ultrasound reflected through a subject in the subject area and generate a second timing signal when a reflected pulse is detected

c) ranging means to assess the time delay between the first and second timing signals from each transmitter/receiver pair, and based on the time delay, to output position data related to the position of a subject in the subject area, and

d) display means to display position data output from the ranging means and/or

e) recording means to record position data output from the ranging means.

The term 'ultrasound beam' as used herein is intended to describe a series of rapid pulses of ultrasound frequency, rather than a continuous emission of ultrasound, and detection of the swaying movement is based on the principle that the time taken for the ultrasonic pulses to travel from the transmitter and return to the receiver after reflection from the subject will vary as the subject sways.

Normally each transmitter/receiver pair is in the form of a single combined unit commonly known as an ultrasonic 'sensor'. In such sensors, the transmitter and receiver are associated with a transducer such that during transmission, the transducer acts as an emitter of ultrasonic pulses. After transmission, the tranducer is switched to operate with the receiver (the transducer being used as a microphone); after receiving reflected ultrasound, the transducer is switched to operate with the transmitter again, and so on.

In normal practice, two sensors are used, each set at right angles to the other. Thus, for human subjects, the sensors may be placed at right-angles, suitably some 700 mm from the body and at a height of approximately 1300 mm. This arangement permits recording of both lateral (sideways) and saggital (front/rear) components of sway).

Suitably the frequencies emitted by the transmitters are in the range 50—70 KHz, either as a 'chirp' (i.e. a burst of pulses comprising several frequencies), or as a pulse of a single frequency. Normally, each transmitter is activated (i.e. caused to emit an ultrasonic pulse or burst of pulses, typically of ca. 1 millisecond duration) approximately 15 times per second. It is desirable that each transmitter be activated alternately in order to avoid false signals by reflection of ultrasound generated by the other transmitter. Outputs from the ranging means may be digital, i.e. pulses whose duration is proportional to the distance swayed, or made available as analogue voltages.

In one embodiment of the invention the deviation of the subject from a fixed 'zero' position may be continuously monitored. Thus the delay between the first and second timing signals is compared with the delay measured when the subject is at a predetermined zero position. In a convenient method of obtaining this comparison, with the subject at the zero position the first timing signal is electronically delayed until it coincides with the second timing signal and the ranging means is adjusted to give a nil output at this coincident position. The swaying of the subject thus causes the second timing signal to be advanced or retarded relative to the first timing signal. The output from the ranging means is related to the advancement or retardation of the second timing signal and is thus directly proportional to the distance of sway from the zero position.

In an alternative, preferred, embodiment of the invention, the deviation of the subject from each sensor is continuously monitored. In this embodiment, the delay between the first and second timing signals may suitably be analysed by a microcomputer and a 'sway index' may be generated which quantifies the swaying movements made by the subject with respect to each sensor over a period of time.

The invention will now be described, by way of example only, with reference to the accompanying drawings in which;

Figure 1 is a system diagram of a sway monitoring device in accordance with the invention;

Figure 2 is a system diagram of a sway monitoring device in accordance with the invention; and

Figure 3 is a system diagram of an integrated micro-processor controlled 'sway index' measurement and display unit.

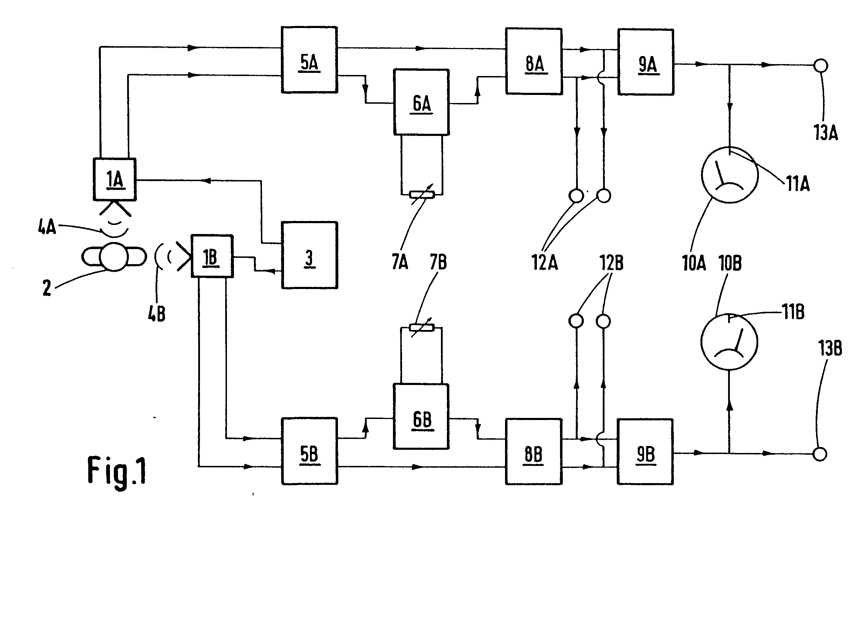

Referring to Figure 1, two ultrasonic sensors 1A (for monitoring saggital sway) and 1B (for monitoring lateral sway) are arranged at right angles at an appropriate distance from a human subject 2. Suitable sensors are commercially available, for example from Polaroid U.K. Ltd. The sensors 1A and 1B are controlled by an alternate drive clock 3 by means of which they may be activated alternately, each sensor being activated approximately 15 times per second. On activation, the sensors 1A and 1B produce a burst of pulses (represented by 'waves' 4A and 4B) at ultrasound frequencies of 50, 53, 57 and 60 KHz. The duration of the pulses produced by each sensor is approximately 1 millisecond.

Immediately the pulses 4A or 4B have been transmitted, a 'sound transmitted' signal pulse is sent from the sensor (1A or 1B) responsible for the transmission to its respective 'sensor interface' (5A or 5B), and the sensor (1A or 1B) is then temporarily switched to become a sensitive receiver using the transducer as a microphone. When an echo from the transmitted pulse (reflected from the subject 2) is detected by sensor 1A or 1B, a logic pulse is generated and fed to the corresponding sensor interface 5A or 5B. The leading edge of such an 'echo-received' signal pulse is used, in conjunction with the leading edge of the 'sound-transmitted' signal pulse, to derive from each sensor interface, 5A and 5B, an output signal proportional to the distance between the corresponding sensor, 1A or 1B, and the subject 2.

When a subject 2 is standing at his or her zero position, variable delay circuits 6A and 6B are adjusted by means of a simple zero control, 7A or 7B, until the 'sound-transmitted' output signal from each sensor interface 5A and 5B is sufficiently retarded for the corresponding 'echo-received' signal to be received simultaneously by respective differential time comparators 8A and 8B. At that point no output pulses are emitted from the differential time comparators 8A and 8B. The differential time comparators 8A and 8B are connected to voltage converters 9A and 9B and thus, in practice, the zero position may be readily defined by adjusting the zero controls 7A and 7B until respective panel meters 10A and 10B show no deflection from median positions 11A and 11B.

A swaying subject 2 will then cause the 'echo' signal to arrive early (when subject 2 moves towards the sensor 1A or 1B) or late (when subject 2 moves away from sensor 1A or 1B) with respect to the 'sound-transmitted' signal. The result of such swaying movement is to cause the differential time caparator 8A or 8B to emit pulses, the width of which is proportional to the distance moved by the subject 2, from the zero position, away from or towards the sensor 1A or 1B.

The digital outputs 12A and 12B from the differential time comparators 8A and 8B may be fed directly to a computer or converted by means of corresponding voltage converters 9A and 9B to output, at points 13A and 13B, analogue voltages that vary linearly with sensor-to-subject distance. These can be recorded independently on a dual channel Yt chart recorder or combined on an X—Y plotter. Such recordings can be immediately visually interpreted for frequency, amplitude of sway and, if desired, recorded on magnetic tape to enable later replay into a computer. Provision of a computer system is advantageous for signal analysis by such methods as the Fourier Transform or 'line-length' evaluation (i.e. determination of the total length of a trace).

Since the absolute sensor-to-subject distance is not determined, it is not necessary to know precisely the velocity of the sound waves that comprise the pulse burst. This velocity is dependent upon the temperature of the air and, to a lesser extent, upon humidity. The formula:

$$\text{velocity} = 331.4 \times \sqrt{T/273}\ \text{ms}^{-1}$$

where T is the air temperature in degrees Kelvin, shows that for measurements taken at room temperature between 10 and 30°C the change in velocity is ± 1.7%. This factor is not compensated for in the device described above.

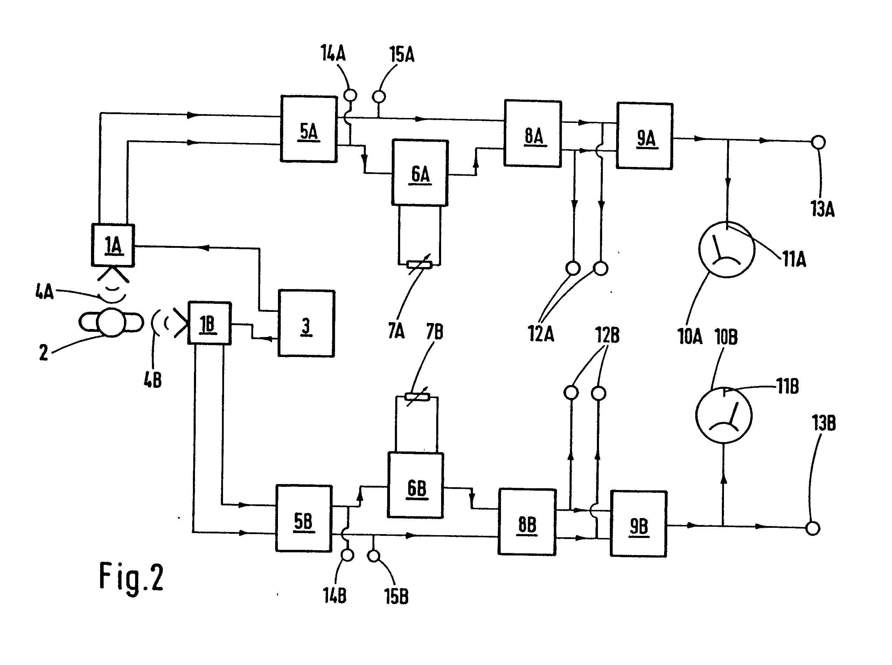

In Figure 2, the arrangement of components is the same as shown in Figure 1 as hereinbefore described, but in addition terminals 14A and 14B and 15A and 15B are provided. From these terminals the output signals from the sensor interfaces 5A and 5B may be fed into a microcomputer programmed to record and display data related to the swaying movements of the subject over any given time period. In this embodiment, the variable delay circuits 6A and 6B, zero controls 7A and 7B, and other components 8A—13A and 8B—13B are optional, but are usefully retained so that, if desired, a chart recorder may be used simultaneously with the microcomputer.

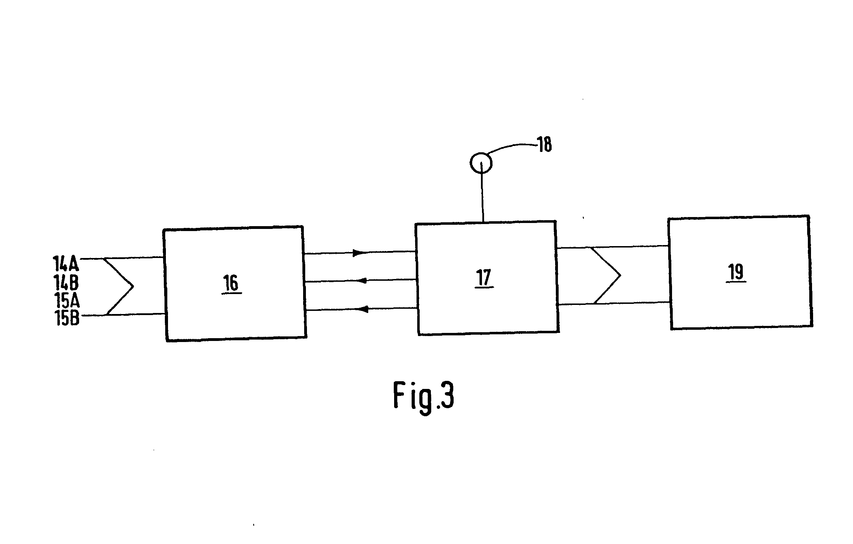

Referring to Figure 3, the signals from the terminals 14A, 14B and 15A, 15B may be fed into a signal conditioning unit 16 which converts each output into a pulse, the duration of which varies with the distance of the subject from the sensors 1A and 1B (Figure 2). The pulses from the signal conditioning unit 16 then pass into a microcomputer 17 which may, for example, be programmed to generate a 'sway index'. The sway index (S) is a numerical value which gives a measure of the swaying motions made by the subject 2 over a given time period and two separate figures — one for lateral sway and one for sagittal sway — are computed.

If (as has been found convenient) it is desired to measure the sway index over a period of 1 minute, the microcomputer 17 is programmed to calculate S according to the equation:

$$S = \sum_{i=1}^{60n} |X_{i+1} - X_i|$$

where n is the number of ultrasonic pulses per second generated by the transmitters 1A and 1B, and $X_i$ is the time between the $i^{th}$ transmitted and received pulse.

The measurement of the sway index may be commenced by manually pressing a start button 18 and at the end of 1 minute, the values of S obtained for both sagittal and lateral sway are displayed on the LCD charter display 19. It will be appreciated that sway index data may be generated in this way without any need for the subject to stand at a pre-determined zero position provided he or she stands within the beam path and within the ranging capabilities of the instrument. The computer is programmed to detect any movement out of the beam path and measurement of the sway index cannot be started or will be discontinued if that occurs.

The application of ultrasound to the measurement of body sway by means of the present invention provides a compact, portable, inexpensive, objective, non-contact method well suited to use in psychopharmacological studies both in normal healthy volunteers and in patients.

## Claims

1. A sway detecting and monitoring device comprising:

a) two or more ultrasound transmitters (IA, IB) arranged to direct a respective ultrasound pulse (4A, 4B) across a subject area amd generate a first timing signal when the pulse is transmitted

b) an ultrasound receiver (IA, IB) associated with each transmitter (IA, IB) to detect ultrasound reflected from a subject (2) in the subject area and generate a second timing signal when a reflected pulse is detected

c) ranging means (5A, 5B) to assess the time delay between the first and second timing signals from each transmitter/receiver pair, and based on the time delay, to output position data related to the position of a subject in the subject (2) area, and

d) display means to display position data output from the ranging means and/or

e) recording means to record position data output from the ranging means.

2. A device as claimed in claim 1 in which each ultrasound transmitter and receiver is in the form of a single combined unit or sensor (IA, IB).

3. A device as claimed in claim 2 in which two sensors [(1A), (1B)] are used, each set at right angles to the other.

4. A device as claimed any one of claims 1 to 3 in which the frequencies emitted by the transmitters (1A, 1B) are in the range of 50—70 KHz.

5. A device as claimed in any one of claims 1 to 4 in which the deviation of the subject from a fixed zero position is continuously monitored.

6. A device as claimed in any one of claims 1 to 4 in which the deviation of the subject from each transmitter is continously monitored.

7. A device as claimed in claim 6 comprising a computer system for analyzing the deviation of the subject from each transmitter (IA, IB).

8. A device as claimed in any one of claims 6 to 8 comprising a computer which calculates a 'sway index' S in accordance with the equation.

$$S = \sum_{i=1}^{60n} |x_{i+1} - x_i|$$

where n is the number of ultrasonic pulses per second generated by the transmitters (1A) and (1B), and $X_i$ is the time between the $i^{th}$ transmitted and received pulse.

## Patentansprüche

1. Vorrichtung zum Ermitteln und Überwachen von Bewegungen, mit

a) mindestens zwei Ultraschall-Sendern (IA, IB) zum Richten eines entsprechenden Ultra-schall-Impulses (4A, 4B) über eine Gegenstandsfläche und Erzeugen eines ersten Zeitsignals, wenn der Impuls gesendet wird;

b) einem Untraschall-Empfänger (IA, IB), der jedem Sender (IA, IB), zugeordnet ist, um Ultraschall, der von einem Gegenstand (2) in der Gegenstansfläche reflektiert wird, festzustellen und eine zweites Zeitsignal zu erseugen, wenn ein reflektierter Impuls festgestellt wird;

c) einer Entfernungsmeßeinrichtung (5A, 5B) zum Ermitteln der Zeitverzögerung zwischen dem ersten und dem zweiten Zeitsignal von jedem Sender/Empfänger-Paar und zum Ausgeben von Positionsdaten auf der Grundlage der Zeitverzögerung, die der Position eines Gegenstandes (2) in der Gegenstandsfläche entsprechen; und mit

d) einer Anzeigeeinrichtung zum Anzeigen der Positionsdatenausgangssignale von der Entfernungsmeßeinrichtung und/oder

e) einer Aufzeichnungseinrichtung zum Aufzeichnen der Positionsdatenausgangssignale von der Entfernungsmeßeinrichtung.

2. Vorrichtung nach Anspruch 1, wobei jeder Ultraschall-Sender und -Empfänger in Form einer einzigen Kombinationseinheit oder eines einzigen Kombinationssensors (IA, IB) vorliegt.

3. Vorrichtung nach Anspruch 2, wobei zwei Sensoren [(IA), (IA)] verwendet werden, die jeweils unter rechten Winkeln zueinander angeordnet sind.

4. Vorrichtung nach einem der Ansprüche 1 bis 3, wobei die von den Sendern (1A, 1B) emittierten Frequenzen im Bereich von 50 bis 70 kHz liegen.

5. Vorrichtung nach einem der Ansprüche 1 bis 4, wobei die Abweichung des Gegenstandes von einem festgelegten Nullpunkt kontinuierlich überwacht wird.

6. Vorrichtung nach einem der Ansprüche 1 bis 4, wobei die Abweichung des Gegenstandes von jedem Sender kontinuierlich überwacht wird.

7. Vorrichtung nach Anspruch 6 mit einem Computersystem zum Analysieren der Abweichung des Gegenstandes von jedem Sender (IA, IB).

8. Vorrichtung nach einem der Ansprüche 6 bis 8 mit einem Computer, der einen "Bewegungsindex" S entsprechend der Gleichung

$$S = \sum_{i=1}^{60n} | X_{i+1} - X_i |$$

berechnet, wobei n = Anzahl der von den Sendern (1A) und (1B) erzeugten Ultraschall-Impulsen pro Sekunden und $X_i$ = Zeit zwischen dem i-ten gesendeten und empfangenen Impuls.

**Revendications**

1. Dispositif pour détecter et surveiller le balancement, caractérisé en ce qu'il comprend:

a) deux ou plusieurs transmetteurs d'ultrasons (IA, IB) disposés de façon à envoyer une impulsion ultrasonore respective (4A, 4B) à travers une zone d'étude et à créer un premier signal de chronométrage lorsque l'impulsion est transmise;

b) un récepteur d'ultrasons (IA, IB) associé à chaque transmetteur (IA, IB) pour détecter les ultrasons réfléchis par un sujet (2) dans la zone d'étude et créer un second signal de chronométrage lorsque l'impulsion réfléchie est détectée;

c) des moyens de repérage (5A, 5B) pour évaluer le décalage entre les premier et second signaux de chronométrage provenant de chaque paire de transmetteur/récepteur et en fonction de ce décalage de temps, fournie des données de position concernant la position d'un sujet dans la zone (2) d'étude; et

d) des moyens d'affichage pour afficher des données de position provenant des moyens de repérage et/ou

e) des moyens d'enregistrement pour enregistrer les données de position provenant des moyens de repérage.

2. Dispositif suivant la revendication 1, caractérisé en ce que chaque transmetteur et récepteur d'ultrasons est sous la forme d'une seule unité combinée ou détecteur (IA, IB).

3. Dispositif suivant la revendication 2, caractérisé en ce qu'on utilise deux détecteurs [(IA, IB)] chaque jeu étant disposé à angle droit l'un par rapport à l'autre.

4. Dispositif suivant l'une quelconque des revendications 1 à 3, caractérisé en ce que les fréquences émises par les transmetteurs (IA, IB) sont dans la gamme de 50—70 KHz.

5. Dispositif suivant l'une quelconque des revendications 1 à 4, caractérisé en ce que l'écart du sujet par rapport à une position zéro déterminée, est surveillé en continu.

6. Dispositif suivant l'une quelconque des revendications 1 à 4, caractérisé en ce que l'écart du sujet par rapport à chaque transmetteur, est surveillé en continu.

7. Dispositif suivant la revendication 6, caractérisé en ce qu'il comprend un système ordinateur pour analyser l'écart du sujet par rapport à chaque transmetteur (IA, IB).

8. Dispositif suivant l'une quelconque des revendications 6 à 8, caractérisé en ce qu'il comprend on ordinateur qui calcule un "indice de balancement" S suivant l'équation:

$$S = \sum_{i=1}^{60n} | X_{i+1} - X_i |$$

dans laquelle n est le nombre d'impulsions ultrasoniques par seconde émises par les transmetteurs (IA) et (IB) et $X_i$ est le temps entre la $i^{\text{ème}}$ impulsion transmise et reçue.

Fig.1

Fig.2

Fig.3